Europäisches Patentamt

European Patent Office

Office européen des brevets

(11) Publication number: **0 384 058**
**A1**

## EUROPEAN PATENT APPLICATION

(21) Application number: 89305958.4

(22) Date of filing: 13.06.89

(51) Int. Cl.⁵: **C12P 21/00, A61K 39/29,**
**//C12N15/51**

(30) Priority: 09.02.89 US 308441

(43) Date of publication of application:
29.08.90 Bulletin 90/35

(84) Designated Contracting States:
DE FR GB NL

(71) Applicant: **DEVELOPMENT CENTER FOR BIOTECHNOLOGY**
**81 Chang Hsing Street**
**Taipei(TW)**

(72) Inventor: **Hsieh, Jih-Han**
**81 Chang Hsing Street**
**Taipei(TW)**
Inventor: **Shih, Shu-Ching**
**3F,No. 15,Lane 64, Rose Road,Sing-Tien**
**Taipei(TW)**

(74) Representative: **Sexton, Jane Helen et al**
**J.A. Kemp & Co. 14 South Square Gray's Inn**
**London WC1R 5EU(GB)**

(54) Isolation of hepatitis B surface antigen from transformed yeast cells.

(57) A method for isolating hepatitis B surface antigen protein particles produced by yeast cells comprises breaking the cells, concentrating the antigens by selective precipitation and post homogenization and further purifying the antigens.

EP 0 384 058 A1

## Isolation of Hepatitis B Surface Antigen from Transformed Yeast cells

### BACKGROUND OF THE INVENTION

The present invention is related to a method for isolating hepatitis B surface antigen (HBsAg) from transformed yeast cells.

It is known that hepatitis B surface antigen (HBsAg) protein can be purified from infected human plasma and used as a vaccine to prevent hepatitis B virus (HBV) infection. HBsAg genes have been cloned and expressed in E. coli. yeast cells and mammalian cells to reduce the dependency on plasma. The synthesis of HBsAg recovered as 22nm particles in yeast cells, e.g. Saccharomyces cerevisiae, has been reported by Valenzuela et al.,Nature, 298:347-350 (1982); by Hitzeman et al.,Nucleic Acid Research, 11:2745-2763 (1983) and by Miyanohara et al., PNAS, 80:1-5 (1983).

Recombinant HBsAg protein particles produced by transformed Saccharomyces cerevisiae (Baker's yeast) have been isolated as described in U.S. Patent No. 4,707,542. Normally, the expressed HBsAg was separated and purified from cell homogenate via the addition of protease inhibitor of phenylmethylsulfonylfluoride (PMSF) and non-ionic detergent polyoxyethylene (9,5)-p-t-octyl-phenol (Triton® X-100) to the cell homogenate. Then, chaotropic agents such as potassium or sodium thiocyanate (KSCN or NaSCN) or urea, can be used to formulate different forms of surface antigen protein aggregates followed by adsorption onto fused silica, borate N-morpholine propanesulfonic acid (MOPS) buffer elution, hydrophobic interaction chromatography on butyl agarose, treatment with polystyrene beads cross-linked with divinylbenzene (Amberlite XAD-2 resin), ultrafiltration, sepharose 6B chromatography as well as immunoaffinity and ECTHAM-cellulose chromatography purification steps.

An improvement on the hydrophobic interaction chromatography using various hydrophobic groups as the fixing carrier was reported in European Patent publication No. 0179485A2. Also, U.S. Patent No. 4,624,918 described a method for the purification of HBsAg from recombinant cell culture via concentration, precipitation, immunoaffinity column, anion exchange and gel permeation chromatography. The present invention is directed to an improved process for isolation of HBsAg using selective polyethlene glycol precipitation and post homogenization.

### SUMMARY OF THE INVENTION

An object of the present invention is to provide an improved method for the separation and recovery of HBsAg protein particles from transformed yeast cells.

A further object of the present invention is to provide a method for extracting HBsAg from cell homogenate without the addition of inhibitors or detergents.

Another object of the present invention is to provide a faster and more economical method for efficient recovery of HBsAg particles in high purity.

In accordance with the present invention, it has been discovered that HBsAg protein particles produced by transformed yeast cells can be selectively recovered from cell homogenate with high yield by the use of selective polyethylene glycol (PEG) precipitation and post homogenization. The PEG used can be easily removed by diafiltration and zonal ultracentrifugation. Post homogenization facilitates mixing and simplifies downstream processing so that the pretreatment operating steps are reduced and the HBsAg protein recovery yield is enhanced. The highly purified HBsAg protein particles can be obtained by further purification.

### DETAILED DESCRIPTION OF THE INVENTION

The present invention provides a method for isolating hepatitis B surface antigen (HBsAg) protein from transformed yeast cells comprising:

(a) breaking and homogenizing said cells to give a cell homogenate;

(b) separating said protein from said cell homogenate by an extraction which comprises a sequential precipitation, by adding from 1 to 10% w/w at a time (weight of PEG to that of the cell homogenate) of polyethylene glycol (PEG), and post homogenization; and

(c) further purifying said HBsAG protein.

Recombinant hepatitis B surface antigen protein particles are synthesized by transformed yeast cells, especially Saccharomyces cerevisiae. The yeast cells are transformed by the recombinant genes plasmid containing DNA molecular coding for HBsAg protein and cultured in a fermentation medium under suitable conditions. The HBsAg genes can be constitutively expressed in the yeast cells.

The expressed yeast cells are repeatedly washed and centrifuged to remove entrained fermentation medium components. The cells are suspended in tris buffer solution in the presence or absence of a detergent such as polyoxyethylene (9,5)p-t-octylphenol (Triton® X-100). Usually, the cells must be suspended in the buffer containing a detergent to enhance the separation of a protein; however, the method according to the present invention can be carried out in the absence of detergent.

The cell suspension is preferably adjusted to an optical density of about 250 to 350 at 600nm and then pumped into a glass-bead homogenizer such as Dyno-Mill at an optimum flow rate and glass bead concentration to ensure maximum breakage of the cells. A cell homogenate is thus obtained.

The obtained cell homogenate is centrifuged. The cell supernatant is subjected to an extraction comprising selective precipitation and post homogenization. The selective precipitation is carried out by sequentially adding from about 1 to 10% w/w of polyethylene glycol (PEG) having a molecular weight of about 2,000 to 10,000 , preferably 6,000 (w/w is referred to the weight of PEG to that of the cell extract). The post homogenization is carried out with a Polytron cell homogenizer (Kinematics). A preferred scheme comprises adding 3% w/w of PEG to the cell extract to precipitate cellular proteins and after centrifugation, adding 8% w/w of PEG to the supernatant of the first precipitation. The resulting precipitate is then subjected to post homogenization. The level of HBsAg (indicated by the activity) can be recovered over 90% in the precipitate after the second PEG precipitation. Another preferred scheme comprises adding 8% w/w of PEG to the cell extract, subjecting to post homogenization followed by the addition of 3% w/w of PEG to the post-homogenized pellet suspension and then adding 8% w/w of PEG to the second PEG treated supernatant.

The sequential addition of PEG facilitates the removal of the cellular proteins and thus the recovery of HBsAg is improved. The sequential precipitation results in about 150 to 200 folds of purification over usual methods. The effect of the sequential precipitation on the recovery of HBsAg is shown in Tables 1-1 and 1-2. Table 1-1 shows the optimum HBsAg recovery is obtained by the addition of 8% w/w of PEG, but the level of the undesired protein is also very high. When HBsAg is recovered by double-step precipitation, the recovery of undesired protein is decreased (shown in Table 1-2). Therefore, the optimum HBsAg recovery is obtained by the procedure comprising the addition of 3% followed by 8% PEG precipitation.

Table 1-1

| Recovery of HBsAg by Single Step Precipitation with Polyethyleneglycol-6000 (PEG-6000) | | | | | |
|---|---|---|---|---|---|
| PEG% | 1% | 2% | 3% | 4% | 8% |
| HBsAg Recovery(%) | 3.9 | 10.3 | 17.1 | 21.2 | 86.5 |
| Undesired Protein(%) | 7.5 | 16.9 | 34.0 | 35.0 | 46.7 |

Table 1-2

| Recovery of HBsAg by Double Step Precipitation with Polyethyleneglycol-6000 (PEG-6000) | | |
|---|---|---|
| PEG% | 1% + 8% | 2% + 8% | 3% + 8% |
| HBsAg Recovery(%) | 86.3 | 85.1 | 83.2 |
| Undesired Protein(%) | 38.6 | 27.1 | 15.3 |

The post homogenization step can also enhance the recovery of HBsAg. The recovery obtained by the post homogenization (shown in Table 2) suggests that the HBsAg protein synthesis and protein particle formation in the yeast cells are in the organelle such as endoplasmic reticulum. The post homogenization facilitates the release of HBsAg particles from precipitated endoplasmic reticulum or microsome.

Table 2

| Enhanced HBsAg Recovery by Post Homogenization | | | |
|---|---|---|---|
| Run No. | Before | Pellet/Buffer | After |
| 1 | 82.8% | 1/5 | 102.3% |
| 2 | 89.6% | 1/4 | 117.8% |
| 3 | 88.5% | 1/3 | 130.5% |

The HBsAg protein particles are partially purified by the above procedures. Further purification steps include sucrose and CsCl gradient ultracentrifugation, process scale continuous zonal ultracentrifugation, size exclusion chromatography and step-wise CsCl gradient ultracentrifugation. The purified HBsAg particles have the same physical and chemical characteristics as those derived from human plasma.

The following examples are offered to aid in understanding of the present invention and are not to be construed as limiting the scope thereof. Unless otherwise indicated, all parts and percentages are by weight.

<u>EXAMPLES</u>

<u>EXAMPLE 1:</u>

Sixty liters of the fermentation broth of the transformed <u>Saccharomyces</u> <u>cerevisiae</u> yeast cells was subjected by batch centrifugation in a Beckman J2-21 centrifuge at 9000g force (8000 rpm) for 5 minutes using a JA-10 rotor (3 liters capacity per batch), and then 6250 grams wet weight (or approximately 1560 grams dry weight) of the cells were harvested. The cell pellet from the centrifuge tube was suspended in TBS 1 buffer solution (10mM Tris-HCl Tris<hydroxymethyl>aminomethane pH7.5, 0.14M NaCl) and repelleted to remove entrained fermentation medium components. The pelleted yeast cells were resuspended in TBS 2 (10mM Tris-HCl pH7.5, 0.5M NaCl, 0.1% Triton® X-100) to a final optical density at 660nm ($OD_{660}$) of 300 and then the cell suspension was pumped into a homogenizer (Dyno-Mill, Willy, Model KDL) at a flow rate of 70 ml/min with a peristaltic pump. The 19,100 grams of cell homogenate was obtained and centrifuged at 12,000 g force (9000 rpm) for 15 min in the J2-21 centrifuge with a total of 15,400 grams supernatant. The hepatitis B surface antigen (HBsAg) concentration in the supernatant was 10 mg/l as measured by Ausria® method (Abbott Radioimmuno Assay) and the total protein concentration analyzed by Lowry method was 28 g/l .

Polyethlene glycol solution (50%) having a molecular weight of 6,000 (PEG-6000) was added to the supernatant at a rate of 150 g/min to a final concentration of 8% w/w to precipitate HBsAg while stirring at 4°C for 16 hours. After centrifugation at 12,000 g for 15 minutes, the 1365g of precipitated material was dissolved by TBS 1 with three times of the original weight and then homogenized the suspension with Polytron homogenizer at 4,500 rpm for 30 seconds, three times at 4°C. The homogenate was treated with PEG-6000 to a final concentration of 3% w/w to precipitate yeast cellular proteins at 4°C for 6 hours. The precipitated protein was removed by centrifugation at 12,000g (9000 rpm) for 15 minutes. The resulting supernatant was again treated with PEG-6000 to a final concentration of 8% w/w to precipitate HBsAg while stirring at 4°C for 16 hours. The second precipitation was treated with centrifugation at 12,000g for 15 minutes. The supernatant was removed and the pellet (approximately 455 grams) was dissolved in TBS 1 with three times the weight and then homogenized by Polytron. The protein suspension was then filtered through 0.22 micron porous membrane. The filtrate was harvested by continuously washing the membrane with TBS 1. The membrane with molecular weight cut off (MWCO) 100,000 (Millipore Pellicon Unit) was used for ultrafiltration to concentrate the filtrate. The retentate was collected (about 2,000 grams).

The retentate was subjected to further treatment to purify HBsAg from yeast cellular proteins. The separation was performed by differential sedimentation through centrifugation. The 1,800 ml of 40% sucrose solution and 1,400 ml of TBS 1 solution were loaded into the ENI (Electro-Nucleonic Inc., K-3 core) ultracentrifuge using peristaltic pump. Two liters of sample was fed into the bottom of the rotor at a constant rate of 10 liters per hour. A sucrose gradient of 2 to 40 percent was formed by dynamic re-orientation of the centrifuge at 35,000 rpm (90,000 g) at 4°C. The fractions were collected with 200ml per fraction after 3 hours of operation.

Fractions were selected by detection of HAU (Hemagglutination Unit), $OD_{280}$ and refractive index, and pooled together. The utilization of ultrafiltration membranes with molecular weight cut-off of 100,000 for concentration and diafiltration to remove sucrose. The resulted solution was adjusted to a volume of 800ml.

The separation of HBsAg particles was performed by cesium chloride (CsCl) gradient ultracentrifugation with densities ranging from 1.10 to 1.25g/cm$^3$. Fractions were selected and concentrated by diafiltration and ultrafiltration (to about 100ml). Further purification of the HBsAg protein particles from yeast protein was carried out by size exclusion chromatography (TSK HW-65). The selected fractions were pooled together and subjected to a step-wise CsCl gradient ultracentrifugation in Beckman zonal rotor (type Ti-15), using the CsCl stepped solutions: a density of 1.30, 1.25, 1.20, 1.10 and starting from 1.15. After ultracentrifugation at 31,000 rpm (100,000 g), 4°C for 44 hours, the selected HBsAg fractions were dialysed to remove CsCl and concentrated by ultrafiltration to obtain the purified HBsAg protein particles.

EXAMPLE 2:

The fermentation yeast cells were harvested and broken according to the procedures as described in Example 1. After centrifugation (12,000g, 15 min) to remove yeast cell debris, PEG-6000 was added to the supernatant to reach a final concentration of 3% w/w and the resulting solution was stirred at 4°C for 6 ·hours and then centrifuged at 4°C (12,000g, 15 min) to remove the precipitate. PEG-6000 solution (50%) was added to the supernatant to reach a final concentration of 8% w/w while stirring at 4°C for 16 hours. After centrifugation, the precipitate was subjected to a post-homogenization using Polytron at 4°C (4,500 rpm, 30 seconds). The homogenate was then purified according to the procedures described in Example 1 to obtain the purified HBsAg protein particles.

**Claims**

1. A method for isolating hepatitis B surface antigen (HBsAg) protein from transformed yeast cells comprising:
(a) breaking and homogenizing said cells to give a cell homogenate;
(b) separating said protein from said cell homogenate by an extraction which comprises a sequential precipitation, by adding from 1 to 10% w/w at a time (weight of PEG to that of the cell homogenate) of polyethylene glycol (PEG), and post homogenization; and
(c) further purifying said HBsAg protein.

2. A method according to claim 1 wherein step (b) comprises adding 3% w/w of PEG to said cell homogenate to precipitate yeast cellular proteins, centrifuging and adding 8% w/w of PEG to the supernatant and subjecting it to post homogenization.

3. A method according to claim 1 wherein step (b) comprises adding 8% w/w of PEG to said cell homogenate, subjecting it to post homogenization, then adding 3% w/w of PEG to said post-homogenized suspension and adding 8% w/w PEG to said second PEG treated supernatant.

4. A method according to claim 1, 2 or 3 wherein said polyethylene glycol for precipitation has a molecular weight of 2,000 to 10,000.

5. A method according to claim 4, wherein said polyethylene glycol has a molecular weight of about 6,000.

6. A method according to claim 1 wherein said post homogenization is carried out by a Polytron cell homogenizer.

7. A method according to any one of the preceding claims wherein said further purification comprises (i) sucrose and CsCl gradient ultracentrifugation, (ii) process scale continuous zonal ultracentrifugation, or (iii) size exclusion chromatography and step-wise CsCl gradient ultracentrifugation.

8. A method according to any one of the preceding claims wherein said HBsAg protein is synthesized in tansformed Saccharomyces cerevisiae.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 168 234 (GENENTECH) * The whole document, especially page 5, lines 9-27; page 7, lines 1-28; claims * | 1-5 | C 12 P 21/00 A 61 K 39/29 // C 12 N 15/51 |
| Y | --- | 7,8 | |
| X | EP-A-0 235 430 (TAKEDA CHEMICAL INDUSTRIES) * Example 16 * | 1,4 | |
| Y | --- | 7,8 | |
| Y | VACCINE, vol. 7, February 1989, pages 60-68, Butterworth & Co. (Publishers) Ltd; B.W. YOUN et al.: "Purification and characterization of pre-S-containing hepatitis B surface antigens produced in recombinant mammalian cell culture" * The whole document * --- | 7 | |
| A | DE-A-3 150 935 (THOMSSEN) --- | | TECHNICAL FIELDS SEARCHED (Int. Cl.5) |
| A | WO-A-8 100 050 (ALPHA THERAPEUTIC CORP.) ----- | | C 12 P A 61 K C 12 N |

The present search report has been drawn up for all claims

| Place of search | Date of completion of the search | Examiner |
|---|---|---|
| THE HAGUE | 30-05-1990 | SKELLY J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.........................................................................

& : member of the same patent family, corresponding document

EPO FORM 1503 03.82 (P0401)